(19) **Europäisches Patentamt**

**European Patent Office**

**Office européen des brevets**

(11) **EP 0 480 245 B1**

(12) **EUROPEAN PATENT SPECIFICATION**

(45) Date of publication and mention
of the grant of the patent:
**31.07.1996 Bulletin 1996/31**

(51) Int Cl.6: **C12N 5/02**, C12M 3/02

(21) Application number: **91116331.9**

(22) Date of filing: **02.12.1986**

(54) **Method of culturing animal cells**

Verfahren zur Züchtung von Tierzellen

Méthode de culture de cellules animales

(84) Designated Contracting States:
**CH DE FR GB LI**

(30) Priority: **06.12.1985 JP 273503/85**

(43) Date of publication of application:
**15.04.1992 Bulletin 1992/16**

(62) Application number of earlier application in
accordance with Art. 76 EPC: **86116718.7**

(73) Proprietor: **TEIJIN LIMITED**
**Osaka-shi Osaka 541 (JP)**

(72) Inventors:
• **Tokashiki, Michiyuki**
**Hachioji-shi, Tokyo (JP)**

• **Hamamoto, Kimihiko**
**Hino-shi, Tokyo (JP)**
• **Ishimaru, Kenji**
**Iwakuni-shi, Yamaguchi-ken (JP)**

(74) Representative: **Kraus, Walter, Dr. et al**
**Patentanwälte Kraus, Weisert & Partner**
**Thomas-Wimmer-Ring 15**
**D-80539 München (DE)**

(56) References cited:
**EP-A- 0 164 813**        **WO-A-82/01563**
**AU-A- 521 867**         **CH-A- 253 466**
**FR-A- 2 513 264**

**Description**

This invention relates to a method of culturing animal cells, and more particularly, to a method of culturing animal cells in suspension.

Cell culture technology is important for the production of antiviral agents such as viral vaccines and interferons or biochemicals such as hormones. The recent production of monoclonal antibodies having the ability to bind to a particular protein as a target relies on the culture of a hybridoma obtained by fusing antibody-producing cells with myeloma cells, and the solution of problems associated with this technique is an industrially important subject.

Heretofore, cell culture has been carried out on a laboratory scale by using a Petri dish, a test tube, a culture bottle, etc.

Some methods and apparatuses for cell culture have recently been suggested. They are roughly classified into anchorage-dependent culture systems and suspension culture systems selected according to the properties of a particular cell to be cultured.

A suspension cell culture method was proposed in which a spinner flask is given an agitating function by a magnetic stirrer or by a vane wheel on a mechanically driven shaft (see U. S. Patents Nos. 2,958,517 and 3,649,465).

Japanese Laid-Open Patent Publication No. 65180/1982 proposed a suspension culture apparatus in which at least one flexible sheet of a relatively large area supported on a rotatable shaft is used as an agitator and the desired gentle agitation is created for a certain kind of feeble cells such as human diploid cells by rotating the agitator sheet and thus causing it to undulate. In cell culture by this apparatus, the cells are cultured in a fixed amount of nutrients, and the growth of the cells stops while they are at a relatively low cell density.

To prevent the growth of cells from stopping at a relatively low cell density and to culture the cells in large quantities at a high density in suspension, there was proposed a so-called perfusion method comprising culturing cells while supplying a makeup culture medium to a culture tank and in the meantime, discharging the spent medium containing a growth-inhibitory substance out of the tank. In performing culture by this method, it is important to separate the spent medium efficiently from living cells in the suspension and discharge the spent medium out of the tank, and thereby to maintain the growth environment for the cells in the tank under optimum conditions. Various filters or other systems have been proposed for the separation of living cells and the spent medium from the suspension. None of them, however, have proved to be entirely satisfactory for industrial pratice because of one or more disadvantages such as the blockage of the filters or the complexity of the structure of the systems.

Japanese Laid-Open Patent Publications Nos. 82083/1984 and 9482/1985 proposed an apparatus for culturing suspended cells at a high density which comprises a culture supernatant discharging tube concurrently functioning as a cell settling tube and a line for addition of a fresh medium so that the cell culture is effected while adding the fresh medium from the line and simultaneously discharging the culture supernatant from the discharge tube.

Generally, suspended animal cells are as small as several microns to several tens of microns in size, and their specific gravity is not much different from that of the culture medium. Hence, in the aforesaid apparatus adapted for separating the suspended cells from the spent medium, the settling area should be increased to settle the cells advantageously. In the apparatuses disclosed in the above two Japanese patent documents, however, the settling area cannot be made so large as is desired.

US-A-4 814 278 and EP 0 191 356 B1, of which inventorship overlaps that of the present application propose an industrial apparatus and an industrial method which are suitable for the culture of cells in large quantities at a high density by the perfusion technique. This apparatus comprises a cell settling zone, an opening for discharging a spent culture medium from the settling zone and an opening for supplying a fresh culture medium to the suspension culture zone, the suspension culture zone and the settling zone being separated by a partition therebetween in a manner to communicate with each other in the lower portion of the settling zone, and the settling zone being formed between the side wall of the cell culture tank and the partition.

Furthermore, AU-A-521 867 discloses a method for the continuous mass in vitro suspension culture of cells. In this known method the following operation is repeated: The culture medium (lymph fluid) in the centrifuge is replaced with a fresh medium while the cell suspension is centrifuged so that the separated cells may be recovered from the centrifuge, and then the cells in the thin layer in the centrifuge are suspended again, and next the resulting suspension is centrifuged so that the cells may be separated from the medium. The role of the above fresh medium is to rinse the separated cells, and to suspend the cells in a thin layer again.

Finally, EP-A-164 813 which is based on a patent application of the patentee, having earlier priority dates but being not published before the priority date of the present patent, discloses a method of cultivating animal or plant cells, which comprises contacting a cultivation liquor having animal or plant cells suspended therein with a liquid fluorocarbon having molecular oxygen dissolved therein while one of the cultivation liquor and the liquid fluorocarbon is present in a continuous phase of the other. Thus, the subject matter of EP-A-164 813 is to use a liquid fluorocarbon in the suspension culturing of cells. In this subject matter, however, the liquid fluorocarbon is used as an oxygen-supplier, and nothing is suggested on its use for centrifugation.

It is an object of this invention to provide a method of culturing animal cells comprising a simple step of separating living animal cells from a suspension culture fluid containing the animal cells.

Another object of this invention is to provide a method of culturing animal cells comprising a step of withdrawing a part of a suspension culture fluid of animal cells from a culture tank and subjecting the withdrawn culture fluid to an operation of separating living animal cells.

Another object of this invention is to provide a method of culturing animal cells, which comprises returning animal cells separated from part of a suspension fluid of animal cells to a culture tank, meanwhile removing the spent culture fluid from which the animal cells have been separated, and introducing a fresh supply of culture fluid to the culture tank in an amount corresponding to the amount of the removed culture fluid.

Another object of this invention is to provide an industrial efficient cultivating method by the perfusion technique in which a large amount of a suspension culture fluid containing animal cells at a high density can be used.

Another object of this invention is to provide a method of culturing animal cells which comprises a step of efficiently separating animal cells which are susceptible to deformation and rupture by an external force unlike microorganisms and yeasts from a culture fluid containing them by using a centrifugal separating device while the animal cells are kept alive.

Another object of this invention is to provide a method of culturing animal cells which comprises a step of performing the aforesaid separation using a centrifugal separating device having a characteristic structure in a sedimentation surface for the animal cells under the action of a centrifugal force.

Another object of this invention is to provide a method of cultivating animal cells which comprises a step of efficiently withdrawing animal cells separated from a centrifugal separating device while they are kept alive.

Further objects and advantages of this invention will become apparent from the following description.

These objects and advantages of this invention are achieved by a method of culturing animal cells, which comprises

(A) subjecting animal cells to suspension culture in a culture tank,

(B) withdrawing a portion of a suspension culture fluid containing the animal cells from the culture tank,

(C) supplying the withdrawn suspension culture fluid to a centrifugal separating device and the centrifugal separating device being operated under the following conditions

$$(1) \qquad \Theta \leq 1.8 \times 10^4,$$

$$(2) \qquad \bar{Z} \times \Theta \leq 1.8 \times 10^6,$$

$$(3) \qquad Q/S.\bar{Z} \leq 5 \times 10^{-3},$$

and

$$(4) \qquad 5 \leq \bar{Z} \leq 2,000$$

wherein

$\Theta$ is the average residence time (seconds) of the animal cells in the centrifugal separating device,

$\bar{Z}$ is a centrifuging effect in the centrifugal separating operation, which is defined by $r\omega^2/g$ in which r is the distance (cm) from the axis of rotation, $\omega$ is the rotating angular speed (radians/sec.), and g is the acceleration of gravity (cm/sec$^2$),

Q is the amount (cm$^3$/s.) of the suspension culture fluid supplied to the centrifugal separating device per unit time, and

S is the sedimentation area (cm$^2$) when the centrifugal force is acting,

(D) withdrawing the separated animal cells from the centrifugal separating device, together with a liquid carrier, by supplying the liquid carrier which

(a) is substantially immiscible with water,

(b) has a higher density than water, and

(c) does not substantially inhibit the growth of the animal cells,

to said centrifugal separating device,

(E) separating animal cells from the liquid carrier, as required, and

(F) recycling at least a portion of the withdrawn animal cells to the culture tank for the step (A).

In a preferred embodiment of the invention the liquid carrier is supplied together with a culture medium.

In another preferred embodiment of the invention the culture medium is a spent medium.

The method of culturing animal cells in accordance with this invention is applicable to the culturing of animal cells in suspension (suspension culture). The suspension culture denotes a method whereby animal cells are cultured while they are floating in an aqueous medium.

The animal cells to which the culturing method of this invention can be applied are those which can grow or proliferate in suspension. They include not only natural animal cells, but also cells modified artificially or by gene manipulation such as hybridoma cells. Or they may be cells derived from lymphatic cells which produce lymphokine, diploid cells producing biologically active substances such as interferon (IFN), or cells producing various monoclonal antibodies.

The present invention is especially suitable for obtaining monoclonal antibodies at high densities by culturing monoclonal antibody-producing cells.

In step (A) of the method of this invention, the animal cells are cultured in suspension in a culture tank. The culture medium used in the suspension culture is an aqueous medium consisting substantially of water. The aqueous medium contains various additives ordinarily used in the culturing of animal cells, for example inorganic salts, vitamins, coenzymes, glucose, amino acids, antibiotics, and growth promoting factors.

Serum may be added to the culture fluid. But a serum-free culture medium may also be used as the culture fluid. The use of the serum-free culture medium is economically advantageous, and therefore desirable.

The culture tank that can be used in this invention at least comprises an opening for feeding animal cells, an opening for introducing a fresh culture medium, a tube for introduction of air, an agitator, and a conduit for withdrawing the suspension culture fluid. Such a culture tank may be an ordinary culture tank.

In step (B), a portion of the suspension culture fluid containing animal cells is withdrawn from the culture tank, for example through a culture fluid withdrawal conduit. The withdrawal of the suspension culture fluid can be carried out continuously or intermittently. Desirably, a fresh supply of the makeup culture medium is introduced into the culture tank continuously or intermittently in an amount corresponding to the amount of the withdrawn suspension culture fluid containing animal cells. As a result, the concentration of substances which inhibit (adversely affect) the growth of the animal cells and consist mainly of metabolites of the animal cells in the suspension culture fluid within the culture tank can be always maintained at a considerably low level. Thus, the density of the grown animal cells in the suspension culture fluid be increased.

The amount of useful metabolites produced with the growth of the animal cells can be increased by an amount corresponding to the increased density of the grown animal cells.

By maintaining the concentration of the substances which inhibit the growth of the animal cells at a low level with a sufficient control, the culturing can be continued while maintaining an increased density of the grown animal cells over an extended period of time, and the useful metabolites can be produced in increased amounts.

The suspension culture fluid containing animal cells which has been withdrawn from the culture tank is then fed to a centrifugal separating device in Step (C) wherein the animal cells are separated.

The withdrawn suspension culture fluid may be fed into the centrifugal separating device at a time in a treatable amount, or continuously in small portions.

Components containing the separated animal cells and the spent culture medium may be taken out continuously from the centrifugal separating device while the centrifugal separating device is rotating, or after the rotation of the centrifugal separating device is stopped.

Step (C) of the method of this invention can be especailly advantageously performed industrially by a method which comprises continuously feeding a fresh suspension culture medium to the centrifugal separating device in small portions and withdrawing the components containing the separated animal cells and the spent culture medium separately but continuously in small portions (to be preferred to as the continuous method); or a method which comprises continuously feeding the fresh suspension culture medium into the centrifugal separating device and continuously withdrawning the separated spent culture medium in small portions, stopping the feeding of the culture fluid after the lapse of a certain period of time, and thereafter withdrawing the components containing the separated animal cells (to be referred to as the intermittent method).

The centrifugal separating device used in practicing step (C) may be a commercially available device. For example, there can be used a two-component simultaneous recovering type continuous centrifuging system of Hitachi (a large-capacity cooling centrifuge 6PR-52 having an SRR5CT rotor or an SRR3CA rotor built therein). In these commercial centrifuges, the space which will be charged with a fluid to be centrifugally separated is disposed cylindrically around the axis of rotation. According to these centirifugal separators, the animal cells centrifugally separated are sedimented substantially uniformly on a cylindrical sedimenting surface.

Investigations of the present inventors have shown that another suitable example of the centrifugal separating device used in step (C) of the method of this invention is a centrifugal separating device comprising

(a) an opening for feeding a suspension culture fluid,
(b) a sedimentation surface having such a structure that the sedimented animal cells can move along the sedimentation surface,
(c) an animal cell gathering portion where the animal cells moved along the sedimentation surface gather,
(d) an opening for withdrawing the animal cells from the animal cell gathering portion and
(e) a mother liquor discharge opening for discharging the mother liquor of culturing from which the animal cells have been separated.

The above centrifugal separating device is characterized by having the sedimentation surface (b) and the animal cell gathering portion (c). The structure of the sedimentation surface defined in (b) means such a structure that the animal cells which have sedimented on the sedimentation surface do not stay there but can move from the sedimented site to another site along the sedimentation surface by a centrifugal force. Such a structure is typified by a sedimentation surface whose distance to the axis of rotation of a rotating rotor having the sedimentation surface is not equal, but gently varies so that it becomes gradually larger from a site at the shortest distance to a site at the longest distance. The highest centrifugal force acts on the site having the largest distance, and the lowest centrifugal surface, on the site having the shortest distance. A centrifugal force of varying intensities between these two extremities acts on sites between them. Hence, even the animal cells which have sedimented at the site having the shortest distance gradually moves toward sites having a larger distance along the sedimentation surface under the action of a centrifugal force, and soon gather at the site having the largest distance. The animal cell gathering portion (c) is provided at a place where the sedimented animal cells no longer move by the centrifugal force.

The sedimentation surface (b) has such a structure that in one section containing the axis of rotation of a rotor, the distance from the sedimentation surface to the axis of rotation gradually changes. Hence, the animal cells gather very efficiently by the centrifugal force in a very small spot where in a section perpendicular to the axis of rotation, the above site having the shortest distance and the above site having the largest distance from the axis of rotation in one section containing the axis of rotation. If the opening for withdrawing the animal cells is provided at this spot, the animal cells can be withdrawn advantageously.

The centrifugal separating device of course further includes an opening for feeding the culture fluid as described in (a) above, the opening for witghdrawing the animal cells and (e) the opening for discharging the culture mother liquor.

The above centrifugal separating device will be described in detail below with reference to the accompanying drawings.

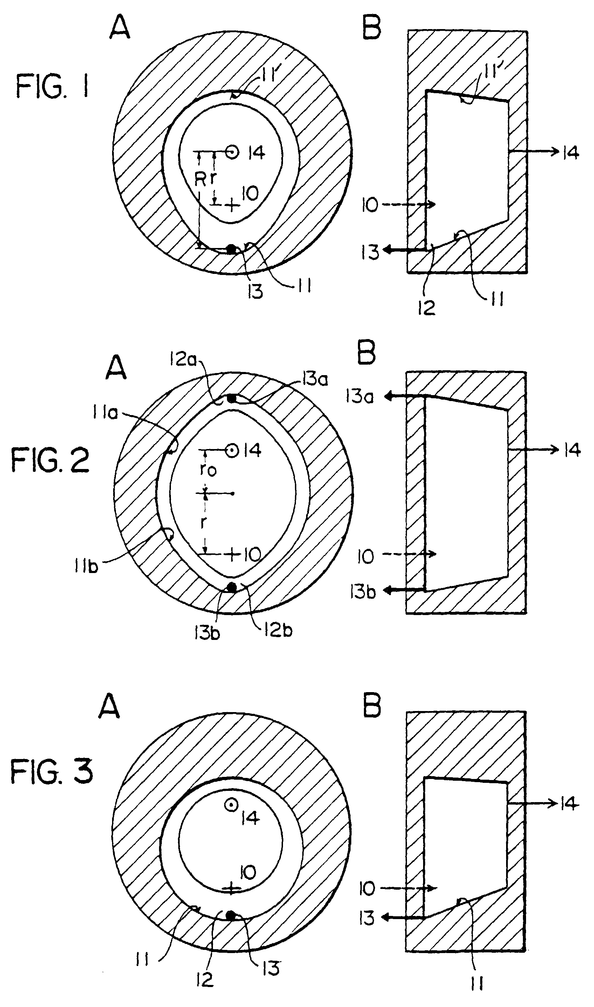

Figures 1, 2 and 3 are views showing the concept of a rotor (separating tank) of a centrifugal separating device suitable for use in this invention. In any of these drawings, A is a sectional view taken in a direction at right angles to the axis of rotation, and B is a sectional view taken in a direction parallel to the axis of rotation and including the axis of rotation.

With reference to Figure 1, the reference numeral 10 represents an opening for feeding the suspension culture fluid to be subjected to separation, and 11 and 11' represent a sedimentation surface for animal cells. The reference numeral 12 represents an animal cell gathering portion; 13, an opening for withdrawing the animal cells which gather in the gathering portion 12; and 14, a mother liquor discharging opening. The opening 10 for feeding the suspension culture fluid is located at a position apart from the axis of rotation of the rotor by a distance $\underline{r}$; and the mother liquor discharge opening 14 is provided at a position corresponding nearly to the axis of rotation of the rotor. In Figure 1, the distance from the axis of rotation of the sedimentation surface of the rotor gradually becomes larger gently from the portion 11' toward the portion 11. Cells which sediment the portion 11' gradually move toward the sedimentation surface by the action of the centrifugal force. As can be well understood from the sedimentation surface is opened from the bottom to the top of the rotor, and the opening at the portion 11 is larger than the opening 11'. By providing such openings, animal cells which sedimented at a site near the top portion of the rotor and animal cells which sedimented at a site near the bottom of the rotor at the portion 11' soon gather at the gathering portion 12. The opening 13 for withdrawal of the animal cells is provided at a site farther from the axis of rotation than the feed opening 10 for the culture fluid by distance R.

The rotor shown in Figure 2 is basically the same as the rotor shown in Figure 1 but differs in the following respects. As can be well seen from Figure 2, A, the rotor has an animal cell sedimentation surface composed of a combination of two sedimentation surfaces 11a and 11b. The two sedimentation surfaces are in such a relation that animal cells sedimented on the sedimentation surface 11a do not substantially move onto the sedimentation surface 11b, and animal cells sedimented on the sedimentation surface 11b do not substantially move on to the sedimentation surface 11a. Hence, the animal cells sedimented onto the sedimentation surface 11a move along the sedimentation surface 11a and arrive at a gathering portion 12a. On the other hand, the animal cells sedimented on the sedimentation surface 11b move along the sedimentation surface 11b and arrive at a gathering portion 12b. There are two openings 13a and 13b for withdrawing the animal cells separated from the gathering portions 12 and 12b. The mother liquor withdrawing opening 14 in the rotor shown in Figure 2 is located at a position apart from the axis of rotation by distance $r_o$. The distance $r_o$ is smaller than the distance r from the axis of rotation to the opening for feeding the suspension culture fluid.

The rotor shown in Figure 3 is basically the same as the rotor shown in Figure 1, but as can be well seen from Figure 3, A, the sedimentation surface 11 is nearly circular. Since, however, the center of this circle deviates from the center of the axis of rotation of the rotor, the animal cells gather at the gathering portion 12 and is withdrawn from the withdrawal opening 13.

The rotors shown in Figures 1, 2 and 3 are suitable when they are operated by the continuous method. The rotor of the centrifugal separating device used in this invention may further comprise a separating plate. The separating plate advantageously catches animal cells at a portion relatively near the axis of rotation, for example, at a portion nearer to the axis of rotation than the culture fluid feed opening, and supplies them to the sedimentation surface.

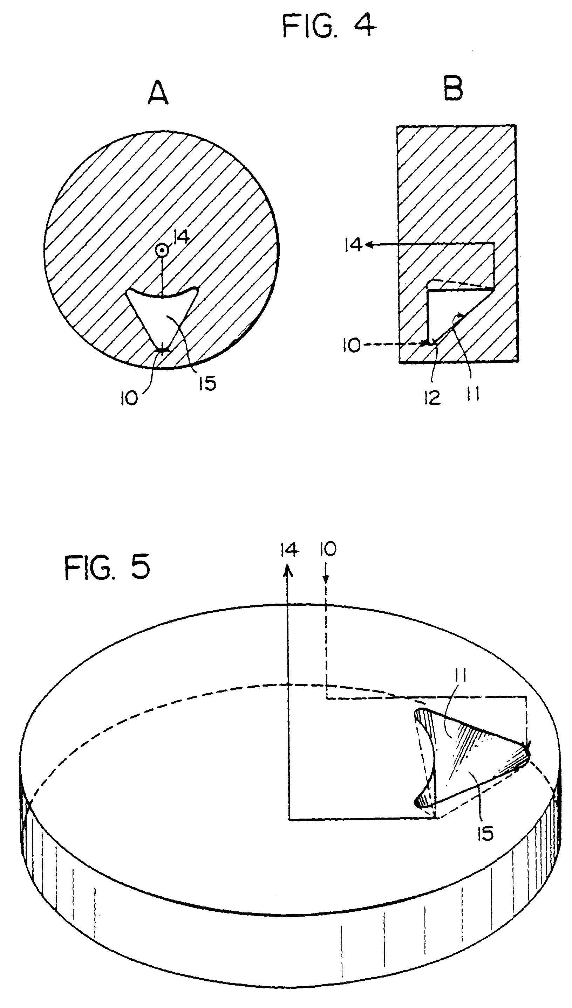

Figure 4 shows another example of the rotor in the centrifugal separating device used in this invention. This rotor is advantageously used to withdraw the separated animal cells intermittently from the centrifugal separating device.

As can be well seen from Figure 4, A and Figure 5, the rotor shown in Figure 4 has a nearly triangular separating tank 15. While the culture fluid is being fed into the separating tank 15 from the culture fluid feed opening 10 for a predetermined period of time, the mother liquor is withdrawn from the mother liquor withdrawing opening 14, and the centrifugal separating device continues to be operated. It will be well seen from Figure 4 that the separating tank 15 has such a sedimentation surface structure that animal cells sedimented gather at the gathering portion 12. The feed opening 10 is provided in the vicinity of the gathering portion 12, and the withdrawing opening 14 is connected to the bottom portion of the separating tank 15. After the centrifugal separating device continues to be operated for a certain period of time as above, the feeding of the culture fluid and the withdrawing of the mother liquor are stopped. The mother liquor is introduced into the separating tank 15 from the withdrawing opening 14, and living cells accumulated in the separating tank are withdrawn together with the mother liquor. During the withdrawal of the animal cells, the rotation of the centrifugal separating device may be stopped if desired.

It will be apparent to those skilled in the art that the aforesaid intermittent operation can also be carried out by the centrifugal separating device having the rotor shown in Figure 1. In this case, the operation of discharging the mother liquor from the mother liquor discharging opening 14 while feeding the culture fluid from the animal cell withdrawing opening is carried out for a certain period of time. Thereafter, the mother liquor is fed to the rotor from the mother liquor discharge opening, and the animal cells accumualted in the rotor are taken out together with the mother liquor from the withdrawal opening 13.

According to the present invention, the centrifugal separating device should be operated under the following operating conditions in order to separate the animal cells efficiently from the culture fluid in which they are suspended while being kept alive. Generally, the animal cells are susceptible to deformation and rupture by an external force. Hence, it is difficult to separate the animal cells efficiently from the culture fluid while they are kept alive. Investigations of the present inventors have shown it is critical that the following operating conditions (1) to (4) should be simultaneously satisfied.

$$(1) \qquad \theta \leqq 300,$$

$$(2) \qquad \bar{Z} \times \theta \leqq 3 \times 10^4,$$

$$(3) \qquad Q/S.\bar{Z} \leqq 0.3,$$

and

$$(4) \qquad 5 \leqq \bar{Z} \leqq 2,000$$

wherein

Q is the average residence time (minutes) of the animal cells in the centrifugal separating device,
$\bar{Z}$ is a centrifuging effect,
Q is the amount (ml/min.) of the suspension culture fluid supplied to the centrifugal separating device per unit time, and
S is the sedimentation area ($cm^2$) when the centrifugal force is acting.

The average residence time ($\theta$, minutes) of the animal cells in the centrifugal separating device should be limited to not more than 300 minutes. If the residence time exceeds 300 minutes, the survival rate of the animal cells becomes markedly low owing, for example, to the deficiency of oxygen. Preferably, the average residene time ($\theta$) is not more than 150 minutes, especially not more than 60 minutes.

For example, in the continuous method comprising continuously feeding the suspension culture fluid into the centrifugal separating device and continuously withdrawing the separated animal cells, the average residence time ($\theta$) of the animal cells in the centrifugal separating device is obtained as a quotient of the volume ($V_A$, cm$^3$) of the space in which the animal cells in the fed suspension culture fluid can exist under centrifugal conditions, divided by the rate ($Q_C$, cm$^3$/min) of withdrawing components containing the separated animal cells from the centrifugal separating device.

$\bar{Z}$ is the centrifugal effect in the centrifugal separating operation, and is represented by $r\omega^2/g$ in which r is the distance (cm) from the axis of rotation, $\omega$ is the rotating angular speed (radians/sec.), and g is the acceleration of gravity (cm/sec$^2$). The centrifuging effect, as it were, represents the magnitude of e centrifugal force exerted on the animal cells, and is therefore determined by the position (distance r from the axis of rotation) of the culture fluid feed opening for feeding the suspension culture fluid to be fed to the centrifugal separating device for separation. $\bar{Z}$ is in the range of 5 to 2,000. If it is lower than 5, the separating operation is difficult to perform. If it exceeds 2,000, the centririfugal force on the animal cells is too high and the cells are undesirably ruptured markedly. Advantageously, the centrifuging effect ($\bar{Z}$) should be maintained in the range of 10 to 1,000, especially preferably 20 to 300.

The $\bar{Z} \times \theta$ should be maintained at not more than $3 \times 10^4$. If the centrifuging operation is carried out while the $\bar{Z} \times \theta$ value is above the above-specified range, the survival rate of the animal cells decreases gradually by the compaction of the cells themselves. Especially preferably, the $\bar{Z} \times \theta$ value is not more than $2 \times 10^4$.

S (cm$^2$) is the sedimentation area (cm$^2$) under the action of a centrifugal force. S (cm$^2$) is defined as an effective area involved in separation at the site (r cm from the axis of rotation) of the opening for feeding the suspension culture fluid into the centrifugal separating device for separation. When a phantom circle at the site of the feed opening at a distance of r from the axis of rotation does not cross the sedimentation surface, it is obtained as a value of $2\pi rh$ by the site (r) of the feed opening and the height (h) of the liquid surface of the suspension culture fluid at the position of the feed opening. When the phantom circle crosses the sedimentation surface, the effective area decreases to the area of that portion which ranges to the site where the phantom circle crosses the sedimentation surface, and is obtained by multiplying $2\pi rh$ by the ratio of the angle to the point of crossing. It should be understood that the height (h) of the liquid surface mentioned above is a vertical distance from the deepest position of the separating tank of the centrifugal separating device.

The value $S.\bar{Z}$ obtained by multiplying the effective area S and the centrifuging effect $\bar{Z}$ is a parameter that shows the separating ability of the centrifugal separating device. In the method of this invention, a value obtained by dividing the amount Q (ml/min.) of the suspension culture fluid supplied to the centrifugal separating device per unit time by this parameter, i.e. $Q/S.\bar{Z}$, should be limited to not more than 0.3, preferably not more than 0.2, especially preferably not more than 0.1.

By ensuring the operating conditions (1) to (4), step (C) of the method of this invention makes it possible to separate living animal cells efficiently at a very high survival rate from the suspension culture fluid.

According to this invention, the separated animal cells are taken out from the centrifugal separating device in step (D). Step (D) can be carried out by continuously withdrawing the separated animal cells in small portions from the centrifugal separating device by the continuous method during operation. Alternatively, it may be carried out by stopping the supplying of the culture fluid in the intermittent method and then withdrawing the separated animal cells.

The animal cells withdrawn while they are alive are partly or wholly returned to the culture tank for suspension culture in step (A). Returning of the animal cells can be conveniently carried out through an animal cell feed opening of the culture tank either continuously or intermittently.

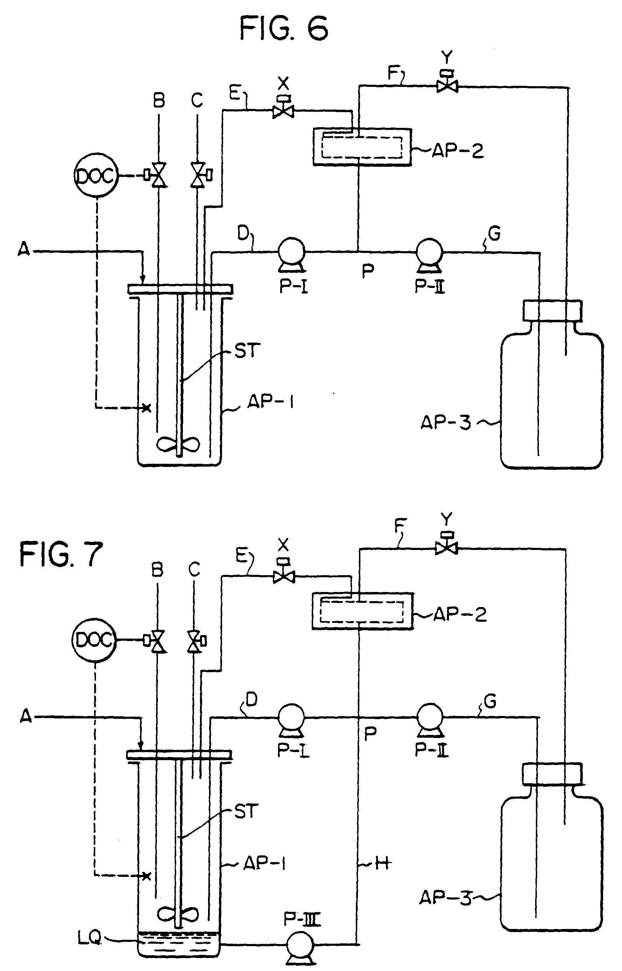

One embodiment of the present invention will be specifically described with reference to Figure 6.

In the apparatus shown in Figure 6, a culture tank AP-1 is provided with a feed opening A for feeding a fresh supply of the culture medium, an air ($O_2$) introducing tube B, a dissolved oxygen controller DOC for measuring the oxygen concentration of the culture fluid in the culture tank and adjusting the oxygen concentration of air to be introduced, a stirrer ST, an air exhaust tube C and a withdrawal conduit D for withdrawing the suspension culture fluid. The withdrawal conduit D extends via a pump P-I and bifurcates into two passages. One passage extends to a spent medium vessel AP-3 via a pump P-II and a conduit G. The other is connected to a centrifugal separator AP02 from which it further extends to the spent medium vessel AP-3 via a conduit F and a valve Y. A conduit E extends from the centrifugal separator AP-2 via a valve X and is connected to the culture tank AP-1.

A fresh supply of the culture medium is fed into the culture tank AP-1 and air is introduced from the air introducing tube B. The stirrer is rotated, and animal cells are seeded in the culture medium to start culturing. When after culturing for a predetermined period of time, the number of cells in the culture tank reached a saturation, rotation of the pump P-1 is started. The culture fluid in the culture tank is sent to the centrifugal separator AP2 via the conduit D, and the mother liquor is separated from the animal cells by the centrifugal separator. The separated mother liquor is continuously sent to the spent medium vessel AP-3 via the conduit F. During this time, a fresh supply of the culture medium is introduced from the feed opening A into the culture tank continuously or intermittently so that the liquid surface of the culture tank AP-1 does not much vary. After the centrifugal separator AP-2 is operated for a certain period of time as above, the operation of the pump P-I is stopped. The valve Y is closed and the valve X is opened. The operation of the pump P-II is then started. Consequently, the mother liquor in the spent medium vessel AP-3 is conducted to the centrifugal separator AP-2 via the conduit G, and while carrying the living animal cells accumulated in AP-2, returned to the culture tank AP-1 via the conduit E. By continuously operating the apparatus in this manner, the concentration of substances which inhibit the growth of the animal cells and are accumulated gradually in the culture tank as the animal cells are cultured can be maintained at a low level. Thus, the method of this invention can be practiced advantageously.

Investigations of the present inventors have shown that animal cells separated from the centrifugal separating device can be withdrawn very smoothly and any possibility of causing damage to the animal cells during withdrawal can be reduced greatly by carrying out the separation of the animal cells from the suspension culture fluid in the centrifugal separating device in the presence of a liquid carrier having the following properties:

(a) it is substantially immisicible with water,
(b) it has a higher density than water, and
(c) it does not substantially inhibit the growth of the animal cells,

while the aforesaid operating conditions are ensured.

Since the liquid carrier is substantially immiscible with water (a) and has a higher density than water (b), a liquid phase of the liquid carrier is formed between the culture fluid and the sedimentation surface in the animal cell gathering portion during the operation of the centrifugal separating device. As a result, the animal cells are prevented from being compacted onto the sedimentation surface, and exist on the liquid phase as a cushion. Thus, at the time of withdrawal, the animal cells move separately from or together with, the liquid carrier. Since the liquid carrier does not substantially inhibit the growth of the animal cells, it can exist together with the animal cells without any problem.

Perfluorocarbons, for example, can be suitably used as the liquid carrier. Advantageously, the fluorocarbons are liquid at room temperatures, and commercially available fluorocarbons can be widely utilized. For example, fluorocarbons used as heat media or an electrically insulating material, and various fluorocarbons used as artificial blood may be used. Specific examples include perfluoroalkanes having at least 8 carbon atoms, perfluorocycloalkanes (such as perfluorodecalin, perfluoromethyldecalin, and perfluoroalkylcyclohexane, and perfluoroalkylcyclohexanes having 3 to 5 carbon atoms in the alkyl moiety), perfluoroalkyltetrahydrofurans having 5 to 7 carbon atoms in the alkyl moiety, perfluoroalkyltetrahydropyrans having 4 to 6 carbon atoms in the alkyl moiety, and perfluoroadamantanes (such as perfluoroadamantane, perfluoromethyladamantane, perfluorodimethyladamantane, perfluoromethylethyladamantane and perfluorodiethyladamantane). These perfluorocarbons may contain various groups, for example a tertiary amino group. They may be used either singly or in combination.

Now, with reference to Figure 7 of the accompanying drawings, the embodiment of this invention using the liquid carrier will be described.

The apparatus of Figure 7 is basically the same as the apparatus of Figure 6, and the same reference letters and numerals have the same meanings.

The difference of the apparatus of Figure 7 from that of Figure 6 is that a conduit H for withdrawing a liquid carrier phase LQ is disposed at the bottom portion of the culture tank AP-1, and the conduit H extends via the pump P-III and is connected at point P to a conduit extending to the centrifugal separating device Ap-2.

In operation, the pump P-III is operated for a short time before the pump P-I is operated to introduce an amount of the liquid carrier LQ into the centrifugal separating device AP-21. Then, the pump P-III is stopped, and the same operation as described with reference to the apparatus of Figure 6 is performed. Since the liquid carrier is partly or wholly introduced into the culture tank AP-1 at the time of withdrawing the separated animal cells from the centrifugal separating device AP-2 through the conduit C, a liquid carrier phase LQ is formed at the bottom of the culture tank. The recyclic use of the liquid carrier phase LQ formed at the bottom of the culture tank is very advantageous to culture on an industrial scale.

According to a preferred embodiment of this invention, the liquid carrier is sent to the centrifugal separating device also at the time of withdrawing the separated animal cells from the centrifugal separating device. In the apparatus shown in Figure 7, the liquid carrier can be sent to the centrifugal separating device by operating the pump P-III simultaneously with the operation of the pump P-II. This can completely prevent the animal cells from being damaged until the animal cells are withdrawn from the centrifugal separating device.

The following examples illustrate the method of this invention in greater detail.

EXAMPLE 1

(1) Culture device

A culture system of the type shown in Figure 6 was used. The culture tank (AP-1) was a glass stirred culture tank having a total capacity of 2 liters and adapted to receive 1.2 liters as a net volume of the culture fluid. AP-2 in Figure 6 is a centrifugal separator with a rotor having an effective volume of 11 ml and a sedimentation area S of 31.4 cm$^2$. Pumps P-I and P-III are peristatic pumps.

(2) Culture medium

A 2:1:1 mixture of RPMI, 1640 medium, Ham-12 medium and Dulbeccos' modified Eagle medium (to be referred to as RDF) was used as a base medium.

A culture medium was prepared by adding 2 micrograms/ml of insulin, 10 micrograms/ml of transferin, 10 micrograms/ml of ethanolamine and 2 x 10$^{-8}$ mole/liter of selenous acid to the base medium.

(3) Method and result of culure

The culture system was sterilized in advance by autoclaving. 1.2 liters of the medium sterilized by filtration was fed into the culture tank, and mouse-mouse hybridoma 4C10B6 cells derived from mouse myeloma P3UI were seeded at a density of 2 x 10$^5$ cells/ml. These hybridoma cells produce IgG. Oxygen gas containing 5% of carbon dioxide gas was introduced into the culture tank through a blow nozzle B while the concentration of dissolved oxygen in the culture fluid was automatically controlled to 3 mm. The culture fluid in the culture tank was maintained at 37°C. A marine-type stirring vane was attached to the cultivation tank, and operated at a stirring speed of 60 rpm.

For four days after seeding, the culturing was carried out batchwise. As shown in Table 1, the cell density reached 1.0 x 10$^6$ cells/ml on the fourth day after starting the culturing. This density was determined to be the maximum density in batch culture, and perfusion culture using a centrifugal separator was then started. The centrifugal separator charged with the filtration-sterilized culture fluid was driven, and the rotation speed of the centrifugal separator was adjusted so that the centrifuging effect became 10G. Then, valve X was closed, pump P-II was stopped, and valve Y was opened. In this state, pump P-I was operated to send 200 ml of the culture fluid to the centrifugal separator for 10 minutes at a rate of 20 ml/min. The mother liquor separated from the cells in the centrifugal separator was sent to the spent medium vessel AP-3 through line F and stored. In the spent medium vessel AP-3, the cell density was 2.4 x 10$^5$ cells/ml. After the lapse of 5 minutes from the termination of the foregoing operation, valve X was opened, valve Y was closed and pump P-I was stopped. In this stage, pump G was operated to send 50 ml of the spent medium to the centrifugal separator from vessel AP-3 at a rate of 10 ml/min. By this operation, all cells residing in the centrifugal rotor were discharged from the centrifugal separator, and returned to the culture tank AP-1 via the line E. After 50 ml of the spent medium was sent, the pump P-II was stopped. The culturing was continued by performing this operation automatically once every period of time indicated in Table 1. A fresh supply of the culture medium was continuously fed into the culture tank from line A so that the liquid level of the culture tank was constant. On the fifth day after the starting of the culturing and thereafter, the culture fluid containing cells was withdrawn from the culture tank at a rate of 120 ml per day. In the above experiment, Q/S.$\bar{Z}$ was 0.064 cm/min.; θ was 12.5 min., and $\bar{Z}$.θ was 125.

Some of the experimental conditions and the results of the experiment are shown in Table 1. In the table, the ratio of the make-up medium means the number of feedings of the makeup medium to be fed per day on the basis of the effective culture volume.

Table 1

| Culturing time (days) | Time interval for feeding to the centrifugal separator (hours) | Number of feedings of the makeup medium per day | Density of living cells (cells/ml) | Concentration of the antibody in the culture fluid ($\mu$g/ml) |
|---|---|---|---|---|
| 0 | - | 0 | 2 x10$^5$ | 0 |
| 1 | - | 0 | 3.1 x 10$^5$ | 4 |
| 2 | - | 0 | 6.1 x 10$^5$ | 11 |
| 3 | - | 0 | 1.0 x 10$^6$ | 28 |
| 4 | 3 | 1.0 | 2.3 x 10$^6$ | 37 |
| 5 | 3 | 1.0 | 3.8 x 10$^6$ | 41 |
| 6 | 2 | 1.5 | 6.4 x 10$^6$ | 53 |
| 7 | 2 | 1.5 | 8.2 x 10$^7$ | 62 |
| 8 | 1.5 | 2.0 | 1.0 x 10$^7$ | 57 |
| 9 | 1.5 | 2.0 | 1.1 x 10$^7$ | 61 |

EXAMPLE 2

(1) Culture apparatus

The same apparatus as in Example 1 was used.

(2) Culture medium

There was used a culture medium obtained by adding 9 micrograms/ml of insulin, 10 micrograms/ml of transferin, 10 micrograms/ml of ethanolamine and 2 x $10^{-5}$ mole/liter of selenous acid to the same base medium as in Example 1.

(3) Method and result of culturing

The culture system was sterilized by autoclaving. Then, 1.2 liters of the culture medium sterilized by filtration was fed into the culture tank. Then, mouse-human hybridoma H-2 cells obtained by fusing mouse myeloma P3UI cells with human B cells were seeded at a density of 5 x $10^5$ cells/ml. These hybridoma cells produce IgG. Oxygen gas containing 5% of carbon dioxide gas was introduced by a blow nozzle B into the culture tank so that the concentration of dissolved oxygen in the culture fluid was maintained at 3 ppm by automatic control. The culture fluid in the culture tank was maintained at 37°C. A marine-type stirring vane was attached to the culture tank, and its stirring speed was 60 rpm.

For 3 days after seeding, batchwise culture was performed. As shown in Table 2, the cell density reached 1.0 x $10^6$/ml on the third day after starting the culture (after the lapse of 24 hours), the cell density reached 1.0 x $10^6$ cells/ml. Since this cell density was determined to be the highest density in batchwise culture, and perfusion culture using a centrifugal separator was started. A centrifugal separator charged with the filtration-sterilized culture fluid was driven, and the rotation speed of the centrifugal separator was adjusted so that the centrifuging effect became 20G. Then, valve X was closed, pump P-II was stopped, and valve Y was opened. In this state, pump P-I was operated to send 200 ml of the culture fluid to the centrifugal separator for 10 minutes at a rate of 20 ml/min. The mother liquor separated from the cells in the centrifugal separator was sent to the spent medium vessel AP-3 through line F and stored. In the spent medium vessel AP-3, the cell density was 1.8 x $10^5$ cells/ml. After the lapse of 5 minutes from the termination of the foregoing operation, valve X was opened, valve Y was closed and pump P-I was stopped. In this state, pump G was driven to send 50 ml of the spent medium to the centrifugal separator from vessel AP-3 at a rate of 10 ml/min. By this operation, the cells residing in the centrifugal rotor were all discharged from the centrifugal separator, and returned to the culture tank AP-1 via the line E. After 50 ml of the spent medium was sent, the pump P-II was stopped. The culturing was continued by performing this operation automatically once every period of time indicated in Table 2. A fresh supply of the culture medium was continuously fed into the culture tank from line A so that the liquid level of the culture tank was constant. On the fourth day after the starting of the culturing and thereafter, the culture fluid containing cells was withdrawn from the culture tank at a rate of 120 ml per day. In the above experiment, $Q/S.\bar{Z}$ was 0.032 cm/min.; $\theta$ was 12.5 mm., and $\bar{Z}.\theta$ was 250.

Some of the experimental conditions and the results of the experiment are shown in Table 2.

Table 2

| Culturing time (days) | Time interval for feeding to the centrifugal separator (minutes) | Number of feedings of the makeup medium per day | Density of living cells (cells/ml) | Concentration of the antibody in the culture fluid (μg/ml) |
|---|---|---|---|---|
| 0 | - | 0 | 5 x$10^5$ | 0 |
| 1 | - | 0 | 7 x $10^5$ | 4 |
| 2 | - | 0 | 1.0 x $10^6$ | 15 |
| 3 | 3 | 1.0 | 1.7 x $10^6$ | 26 |
| 4 | 2 | 1.5 | 3.0 x $10^6$ | 21 |
| 5 | 1.5 | 2.0 | 4.9 x $10^6$ | 22 |
| 6 | 1.0 | 3.0 | 7.8 x $10^6$ | 28 |
| 7 | 0.5 | 6.0 | 1.1 x $10^7$ | 19 |
| 8 | 0.5 | 6.0 | 1.3 x $10^7$ | 25 |
| 9 | 0.5 | 6.0 | 1.1 x $10^7$ | 31 |
| 10 | 0.5 | 6.0 | 1.4 x $10^7$ | 29 |

EXAMPLE 3

(1) Culture apparatus

The same apparatus as in Example 1 was used.

(2) Culture medium

The same culture medium as in Example 2 was used.

(3) Method and result of culturing

The culture system was sterilized by autoclaving. Then, 1.2 liters of the culture medium sterilized by filtration was fed into the culture tank. Then, mouse-mouse hybridoma V-6 cells obtained by fusing mouse myeloma P3UI cells with human B cells were seeded at a density of $5 \times 10^5$ cells/ml. These hybridoma cells produce IgG. Oxygen gas containing 5% of carbon dioxide gas was introduced by a blow nozzle B into the culture tank so that the concentration of dissolved oxygen in the culture fluid was maintained at 3 ppm by automatic control. The culture fluid in the culture tank was maintained at 37°C. A marine-type stirring vane was attached to the culture tank, and its stirring speed was 60 rpm.

For 3 days after seeding, batchwise culture was performed. As shown in Table 3, the cell density reached $1.1 \times 10^6$/ml on the third day after starting the culture. Since this cell density was determined to be the highest density in batchwise culture, and perfusion culture using a centrifugal separator was started. A centrifugal separator charged with the filtration-sterilized culture fluid was driven, and the rotation speed of the centrifugal separator was adjusted so that the centrifuging effect became 50G. The valve X was closed, pump P-II was stopped, and valve Y was opened. In this state, pump P-I was operated to send 200 ml of the culture fluid to the centrifugal separator for 4 minutes at a rate of 50 ml/min. The mother liquor separated from the cells in the centrifugal separator was sent to the spent medium vessel AP-3 through line F and stored. In the spent medium vessel AP-3, the cell density was $0.7 \times 10^5$ cells/ml. After the lapse of 5 minutes from the termination of the foregoing operation, valve X was opened, valve Y was closed and pump P-I was stopped. In this state, 50 ml of the spent medium was sent to the centrifugal separator at a rate of 10 ml/min. By this operation, the cells residing in the centrifugal rotor were all discharged from the centrifugal separator, and returned to the culture tank AP-1 via the line E. After 50 ml of the spent medium was sent, the pump P-II was stopped. The culturing was continued by performing this operation automatically once every period of time indicated in Table 3. A fresh supply of the culture medium was continuously fed into the culture tank from line A so that the liquid level of the culture tank was constant. On the fourth day after the starting of the culturing and thereafter, the culture fluid containing cells was withdrawn from the culture tank at a rate of 120 ml per day. In the above experiment, $Q/S.\bar{Z}$ was 0.032 cm/min.; $\theta$ was 4.5 mm., and $\bar{Z}.\theta$ was 225.

Some of the experimental conditions and the results of the experiment are shown in Table 3.

Table 3

| Culturing time (days) | Time interval for feeding to the centrifugal separator (minutes) | Number of feedings of the makeup medium per day | Density of living cells (cells/ml) | Concentration of the antibody in the culture fluid (µg/ml) |
|---|---|---|---|---|
| 0 | - | 0 | $5 \times 10^5$ | 0 |
| 1 | - | 0 | $7.1 \times 10^5$ | 2 |
| 2 | - | 0 | $1.1 \times 10^6$ | 6 |
| 3 | 90 | 2 | $1.8 \times 10^6$ | 5 |
| 4 | 90 | 2 | $2.9 \times 10^6$ | 7 |
| 5 | 45 | 4 | $3.9 \times 10^6$ | 6 |
| 6 | 45 | 4 | $7.1 \times 10^6$ | 9 |
| 7 | 45 | 4 | $9.2 \times 10^6$ | 10 |
| 8 | 33 | 5.5 | $1.2 \times 10^6$ | 9 |
| 9 | 33 | 5.5 | $1.3 \times 10^6$ | 11 |

EXAMPLE 4

(1) Culture apparatus

The same apparatus as in Example 1 was used.

(2) Culture medium

The same culture medium as in Example 2 was used.

(3) Method and result of culturing

The culture system was sterilized by autoclaving. Then, 1.2 liters of the culture medium sterilized by filtration was fed into the culture tank. Then, mouse-human hybridoma H-2 cells were seeded at a density of $5 \times 10^5$ cells/ml. These hybridoma cells produce IgG. Oxygen gas containing 5% of carbon dioxide gas was introduced by a blow nozzle B into the culture tank so that the concentration of dissolved oxygen in the culture fluid was maintained at 3 ppm by automatic control. The culture fluid in the culture tank was maintained at 37°C. A marine-type stirring vane was attached to the culture tank, and its stirring speed was 60 rpm.

For 3 days after seeding, batchwise culture was performed. As shown in Table 4, the cell density reached 0.9 x $10^6$/ml on the third day after starting the culture. Since this cell density was determined to be the highest density in batchwise culture, and perfusion culture using a centrifugal separator was started. A centrifugal separator charged with the filtration-sterilized culture fluid was driven, and the rotation speed of the centrifugal separator was adjusted so that the centrifuging effect became 100G. The valve X was closed, pump P-II was stopped, and valve Y was opened. In this state, pump P-I was operated to send 200 ml of the culture fluid to the centrifugal separator for 2 minutes at a rate of 100 ml/min. The mother liquor separated from the cells in the centrifugal separator was sent to the spent medium vessel AP-3 through line F and stored. In the spent medium vessel AP-3, no cell was seen to exist. After the termination of the foregoing operation, valve X was opened, valve Y was closed and pump P-I was stopped. In this state, pump G was driven to send 50 ml of the spent medium to the centrifugal separator at a rate of 25 ml/min. By this operation, the cells residing in the centrifugal rotor were all discharged from the centrifugal separator, and returned to the culture tank AP-1 via the line E. After 50 ml of the spent medium was sent, the pump P-II was stopped. The culturing was continued by performing this operation automatically once every period of time indicated in Table 4. A fresh supply of the culture medium was continuously fed into the culture tank from line A so that the liquid level of the culture tank was constant. On the fourth day after the starting of the culturing and thereafter, the culture fluid containing cells was withdrawn from the culture tank at a rate of 120 ml per day. In the above experiment, $Q/S.\bar{Z}$ was 0.032 cm/min.; $\theta$ was 2 mm., and $\bar{Z}$. $\theta$ was 200.

Some of the experimental conditions and the results of the experiment are shown in Table 4.

Table 4

| Culturing time (days) | Time interval for feeding to the centrifugal separator (minutes) | Number of feedings of the makeup medium per day | Density of living cells (cells/ml) | Concentration of the antibody in the culture fluid (µg/ml) |
|---|---|---|---|---|
| 0 | - | 0 | $5 \times 10^5$ | 0 |
| 1 | - | 0 | $7.1 \times 10^5$ | 4 |
| 2 | - | 0 | $9 \times 10^5$ | 14 |
| 3 | 95 | 1.9 | $1.7 \times 10^6$ | 19 |
| 4 | 45 | 4 | $3.1 \times 10^6$ | 14 |
| 5 | 45 | 4 | $6.1 \times 10^6$ | 17 |
| 6 | 32 | 5.6 | $8.3 \times 10^6$ | 21 |
| 7 | 26 | 7 | $9.8 \times 10^6$ | 19 |
| 8 | 26 | 7 | $1.3 \times 10^7$ | 21 |
| 9 | 26 | 7 | $1.4 \times 10^7$ | 24 |
| 10 | 26 | 7 | $1.4 \times 10^7$ | 23 |

EXAMPLE 5

(1) Culture apparatus

The same apparatus as in Example 1 was used.

(2) Culture medium

The same culture medium as in Example 2 was used.

(3) Method and result of culturing

The culture system was sterilized by autoclaving. Then, 1.2 liters of the culture medium sterilized by filtration was fed into the culture tank. Then, mouse-human hybridoma 4C10B6 cells were seeded at a density of $2 \times 10^5$ cells/ml. Oxygen gas containing 5% of carbon dioxide gas was introduced by a blow nozzle B into the culture tank so that the concentration of dissolved oxygen in the culture fluid was maintained at 3 ppm by automatic control. The culture fluid in the culture tank was maintained at 37°C. A marine-type stirring vane was attached to the culture tank, and its stirring speed was 60 rpm.

For 4 days after seeding, batchwise culture was performed. As shown in Table 5, the cell density reached $0.89 \times 10^6$/ml on the third day after starting the culture. Since this cell density was determined to be the highest density in batchwise culture, and perfusion culture using a centrifugal separator was started. A centrifugal separator charged with the filtration-sterilized culture fluid was driven, and the rotation speed of the centrifugal separator was adjusted so that the centrifuging effect became 500G. The valve X was closed, pump P-II was stopped, and valve Y was opened. In this state, pump P-I was operated to send 200 ml of the culture fluid to the centrifugal separator for 2 minutes at a rate of 100 ml/min. The mother liquor separated from the cells in the centrifugal separator was sent to the spent medium vessel AP-3 through line F and stored. In the spent medium vessel AP-3, no cell was seen to exist. After the termination of the foregoing operation, valve X was opened, valve Y was closed and pump P-I was stopped. In this state, pump G was driven to send 50 ml of the spent medium to the centrifugal separator at a rate of 25 ml/min. By this operation, the cells residing in the centrifugal rotor were all discharged from the centrifugal separator, and returned to the culture tank AP-1 via the line E. After 50 ml of the spent medium was sent, the pump P-II was stopped. The culturing was continued by performing this operation automatically once every period of time indicated in Table 5. A fresh supply of the culture medium was continuously fed into the culture tank from line A so that the liquid level of the culture tank was constant. On the fifth day after the starting of the culturing and thereafter, the culture fluid containing cells was withdrawn from the culture tank at a rate of 120 ml per day. In the above experiment, $Q/S.\bar{Z}$ was 0.006 cm/min.; $\theta$ was 2 mm., and $\bar{Z}$. $\theta$ was 1000.

Some of the experimental conditions and the results of the experiment are shown in Table 5.

Table 5

| Culturing time (days) | Time interval for feeding to the centrifugal separator (minutes) | Number of feedings of the makeup medium per day | Density of living cells (cells/ml) | Concentration of the antibody in the culture fluid (μg/ml) |
|---|---|---|---|---|
| 0 | - | 0 | $5 \times 10^5$ | 0 |
| 1 | - | 0 | $6.8 \times 10^5$ | 5 |
| 2 | - | 0 | $8.9 \times 10^5$ | 13 |
| 3 | 95 | 1.9 | $1.7 \times 10^6$ | 21 |
| 4 | 45 | 4 | $3.0 \times 10^6$ | 15 |
| 5 | 45 | 4 | $5.9 \times 10^6$ | 18 |
| 6 | 32 | 5.6 | $8.4 \times 10^6$ | 24 |
| 7 | 26 | 7 | $9.6 \times 10^6$ | 23 |
| 8 | 26 | 7 | $1.4 \times 10^7$ | 28 |
| 9 | 26 | 7 | $1.3 \times 10^7$ | 21 |
| 10 | 26 | 7 | $1.5 \times 10^7$ | 24 |

COMPARATIVE EXAMPLE 1

Mouse-mouse hybridoma 4C10B6 cells were cultured under the same conditions except as noted below.
Centrifuging effect: 2,500 G
Rate of sending 200 ml of the culture fluid to the centrifugal separator: 100 ml/min. (for 2 minutes)
After sending the culture fluid as above, the operation of withdrawing the cells was immediately started.
Sending of a cell-free culture medium for withdrawing the cells from the centrifugal separator:
Amount of the medium sent: 50 ml
Rate of sending the medium: 25 ml/min.
In the above experiment, $Q/S.\bar{Z}$ was 0.0016 cm/min., $\theta$ was 2 mm, and $\bar{Z}.\theta$ was 5,000.
Some of the experimental conditions and the results of the experiment are shown in Table 6.
The cells were greatly damaged as a result of passage through the centrifugal separator, and the density of the living cells decreased with time. Accordingly, after the lapse of 5 days, the culturing was stopped.

Table 6

| Culturing time (days) | Time interval for feeding to the centrifugal separator (hours) | Number of feedings of the makeup medium per day | Density of living cells (cells/ml) | Concentration of the antibody in the culture fluid ($\mu$g/ml) |
|---|---|---|---|---|
| 0 | - | 0 | $2 \times 10^5$ | 0 |
| 1 | - | 0 | $2.9 \times 10^5$ | 5 |
| 2 | - | 0 | $5.9 \times 10^5$ | 10 |
| 3 | - | 0 | $9.7 \times 10^5$ | 29 |
| 4 | 3 | 1.0 | $7.9 \times 10^5$ | 21 |
| 5 | 3 | 1.0 | $4.2 \times 10^5$ | 13 |

COMPARATIVE EXAMPLE 2

Mouse-human hybridoma H-2 cells were cultured under the same conditions as in Example 2 except as noted below.
Centrifuging effect: 30 G
Amount of the culture fluid sent to the centrifugal separator per cycle: 350 ml
Rate of sending the culture fluid: 20 ml/min.
Time required to end the culture fluid: 17.5 min.
Time which elapsed until the withdrawal of the cells from the centrifugal separator was started after 350 ml of the culture fluid was sent to the centrifugal separator: 5.5 hours
In the above experiment, $Q/S.\bar{Z}$ was 0.031 cm/min., $\theta$ was 341 mm, and $\bar{Z}.\theta$ was 10238.
After sending of the culture fluid to the centrifugal separator was started, no increase in the density of living cells was noted. Therefore, the culturing was stopped after 7 days.
Some of the experimental conditions and the results of the experiment are shown in Table 7.

Table 7

| Culturing time (days) | Time interval for feeding to the centrifugal separator (hours) | Number of feedings of the makeup medium per day | Density of living cells (cells/ml) | Concentration of the antibody in the culture fluid ($\mu$g/ml) |
|---|---|---|---|---|
| 0 | - | 0 | $5 \times 10^5$ | 0 |
| 1 | - | 0 | $7 \times 10^5$ | 6 |
| 2 | - | 0 | $1.1 \times 10^6$ | 17 |
| 3 | 6 | 1.0 | $9.3 \times 10^5$ | 19 |
| 4 | 6 | 1.0 | $7.6 \times 10^5$ | 15 |
| 5 | 6 | 1.0 | $7.2 \times 10^5$ | 14 |
| 6 | 6 | 1.0 | $4.4 \times 10^5$ | 18 |
| 7 | 6 | 1.0 | $6.9 \times 10^5$ | 12 |

COMPARATIVE EXAMPLE 3

Mouse-human hybridoma H-2 cells were cultured under the same conditions as in Example 2 except as noted below.

Centrifuging effect: 1700 G

Amount of the culture fluid sent to the centrifugal separator per cycle: 350 ml

Time which elapsed until the withdrawal of the cells from the centrifugal separator was started after 200 ml of the culture fluid was sent to the centrifugal separator: 12.5 minutes

In the above experiment, $Q/S.\bar{Z}$ was 0.00037 cm/min., $\theta$ was 20 mm, and $\bar{Z}.\theta$ was $3.4 \times 10^4$.

Some of the experimental conditions and the results of the experiment are shown in Table 8.

After sending of the culture fluid to the centrifugal separator was started, no increase in the density of living cells was noted. Therefore, the culturing was stopped after 7 days.

Table 8

| Culturing time (days) | Time interval for feeding to the centrifugal separator (hours) | Number of feedings of the makeup medium per day | Density of living cells (cells/ml) | Concentration of the antibody in the culture fluid ($\mu$g/ml) |
|---|---|---|---|---|
| 0 | - | 0 | $5 \times 10^5$ | 0 |
| 1 | - | 0 | $8 \times 10^5$ | 5 |
| 2 | - | 0 | $1.1 \times 10^6$ | 16 |
| 3 | 3 | 1.0 | $8.1 \times 10^5$ | 21 |
| 4 | 3 | 1.0 | $7.1 \times 10^5$ | 15 |
| 5 | 3 | 1.0 | $6.2 \times 10^5$ | 13 |
| 6 | 3 | 1.0 | $5.5 \times 10^5$ | 15 |
| 7 | 3 | 1.0 | $4.7 \times 10^5$ | 11 |

EXAMPLE 6

(1) Culture apparatus

A culture system of the type shown in Figure 7 was used. The culture tank AP-1 was a glass stirred culture tank having a total volume of 2 liters and adapted to receive 1.2 liters as the net amount of a culture medium to be fed.

In Figure 7, AP-2 was a centrifugal separator with a rotor which had a sedimentation area S of 31.4 $cm^2$ and an effective capacity of 11 ml, and pumps P-I, P-II and P-III were peristatic pumps.

(2) Culture medium

The same culture medium as in Example 2 was used.

(3) Method and result of culturing

The culture system was sterilized by autoclaving. Then, 1.2 liters of the culture medium sterilized by filtration was fed into the culture tank. Mouse-human hybridoma H-2 cells obtained by fusing mouse myeloma P3UI cells and human B cells were seeded at a density of $5 \times 10^6$ cells/ml. The hybridoma cells produce IgG. Oxygen gas containing 5% of carbon dioxide gas was introduced into the culture tank by a blow nozzle B so that the concentration of dissolved oxygen in the culture medium was maintained at 3 ppm by automatic control. The culture fluid in the culture tank was maintained at 37ºC. A marine-type stirring vane was attached to the culture tank, and the rate of stirring was 60 rpm.

For 3 days after seeding, the culturing was carried out batchwise. As shown in Table 9, the cell density reached $1.0 \times 10^6$ cells/ml on the third day after the stark of the culturing (after the lapse of 24 hours). This density was determined to be the highest density attainable in batchwise culture, and perfusion culture was started using a centrifugal separator. The centrifugal separator charged with the culture fluid sterilized by filtration was driven to adjust the rotating speed so as to provide a centrifuging effect of 100 g. Then, valve X was closed, pumps P-II and P-III were stopped, and valve Y was opened. In this state, the pump P-1 was driven, and 200 ml of the culture fluid was sent to the centrifugal separator for 10 minutes at a rate of 20 ml/min. The mother liquor separated from the cells by the centrifugal separator was sent to spent medium vessel AP-3 through line F and stored there. After the lapse of 5 minutes from the end of the above operation, valve X was opened, valve Y was closed, and pump P-I was stopped. In this state, pump P-II was driven, and 50 ml of the spent medium was sent to the centrifugal separaors (AP-2) from vessel AP-3 at a rate of 10 ml/min. Pump P-III was riven simultaneously with the driving of pump P-II to send 5 ml of a fluorocarbon in the lower portion of the culture ank AP-1 was sent at a rate of 1 ml/min. By this operattion, all the cells residing in the rotor of the centrifugal separator were discharged from the centrifugal separator together with the fluorocarbon, and returned to the culture tank AP-1. After 50 ml of the culture fluid was sent the pump P-II was stopped. Furthermore, after 5 ml of the fluorocarbon was sent, the pump P-III was stopped. This culturing was continued by performing this operation automatically every period of time indicated in Table 9. A fresh supply of the culture medium was continuously fed from line A so that the liquid level of the culture tank became constant. On the fourth day after the starring of the culturing and thereafter, 120 ml of the culture fluid was withdraw from the culture fluid per day.

In the above experiment. $Q/S.\bar{Z}$ was 0.032/min., $\theta$ was 12.5 mm, and $\bar{Z}.\theta$ was 250.

Some of the experimental conditions and the results of the experiment were shown in Table 9.

Table 9

| Culturing time (days) | Time interval for feeding to the centrifugal separator (hours) | Number of feedings of the makeup medium per day | Density of living cells (cells/ml) | Concentration of the antibody in the culture fluid ($\mu$g/ml) |
|---|---|---|---|---|
| 0 | - | 0 | $5 \times 10^5$ | 0 |
| 1 | - | 0 | $7 \times 10^5$ | 4 |
| 2 | - | 0 | $1.0 \times 10^6$ | 15 |
| 3 | 3 | 1.0 | $1.7 \times 10^6$ | 26 |
| 4 | 2 | 1.5 | $3.0 \times 10^6$ | 21 |
| 5 | 1.5 | 2.0 | $4.9 \times 10^6$ | 22 |
| 6 | 1.0 | 3.0 | $7.8 \times 10^6$ | 28 |
| 7 | 0.5 | 6.0 | $1.1 \times 10^7$ | 19 |
| 8 | 0.5 | 6.0 | $1.3 \times 10^7$ | 25 |
| 9 | 0.5 | 6.0 | $1.1 \times 10^7$ | 31 |
| 10 | 0.5 | 6.0 | $1.4 \times 10^7$ | 29 |

COMPARATIVE EXAMPLE 4

Mouse-human hybridoma H-2 cells were cultured under the same conditions as in Example 6 except that no fluorocarbon was sent to the centrifugal separator.

The living cells became pelletized in the centrifugal separator, and did not return completely to the culture tank. Thus, no increase in the density of living cells was noted. Hence, the culturing was stopped after the lapse of 7 days.

Some of the experimental conditions and the results of the experiment are shown in Table 10.

Table 10

| Culturing time (days) | Time interval for feeding to the centrifugal separator (hours) | Number of feedings of the makeup medium per day | Density of living cells (cells/ml) | Concentration of the antibody in the culture fluid ($\mu$g/ml) |
|---|---|---|---|---|
| 0 | - | 0 | $5 \times 10^5$ | 0 |
| 1 | - | 0 | $8 \times 10^5$ | 5 |
| 2 | - | 0 | $1.1 \times 10^6$ | 16 |
| 3 | 3 | 1.0 | $8.1 \times 10^5$ | 21 |
| 4 | 3 | 1.0 | $7.1 \times 10^5$ | 15 |
| 5 | 3 | 1.0 | $6.2 \times 10^5$ | 13 |
| 6 | 3 | 1.0 | $5.5 \times 10^5$ | 15 |
| 7 | 3 | 1.0 | $4.7 \times 10^5$ | 11 |

EXAMPLE 7

(1) Culture apparatus

The same culture apparatus as in Example 1 was used

(2) Culture medium

The same culture medium as in Example 1 was used.

Method and result of culturing The culture system was sterilized by autoclaving. Then, 1.2 liters of the culture medium sterilized by filtration was fed into the culture tank. Mouse-mouse hybridoma JTC-3 cells were then seeded at a density of $2 \times 10^5$ cells/ml. These hybridoma cells produce IgG. Oxygen gas containing 5% of carbon dioxide gas was introduced by a blow nozzle B into the culture tank so that the concentration of dissolved oxygen in the culture fluid was maintained at 3 ppm by automatic control. The culture fluid in the culture tank was maintained at 37°C. A marine-type stirring vane was attached to the culture tank, and its stirring speed was 60 rpm.

For 4 days after seeding, batchwise culture was performed. As shown in Table 11, the cell density reached $1.1 \times 10^6$/ml on the third day after starting the culture. Since this cell density was determined to be the highest density in batchwise culture, and perfusion culture using a centrifugal separator was started. A centrifugal separator charged with the filtration-sterilized culture fluid was driven, and the rotation speed of the centrifugal separator was adjusted so that the centrifuging effect became 80G. The valve X was closed, pump P-II was stopped, and valve Y was opened. In this state, pump P-I was operated to send 200 ml of the culture fluid to the centrifugal separator for 30 minutes at a rate of 16.7 ml/min. The mother liquor separated from the cells in the centrifugal separator was sent to the spent medium vessel AP-3 through line F and stored. In the spent medium vessel AP-3, the cell density was $2.4 \times 10^5$ cells/ml. Immediately after the termination of the foregoing operation, valve X was opened, valve was closed and pump P-I was stopped. In this state, pump G was driven to send 55 ml of the spent medium to the centrifugal separator at a rate of 11 ml/min. from the vessel AP-3. By this operation, all cells residing in the centrifugal rotor were discharged from the centrifugal separator, and returned to the culture tank AP-1 via the line E. After 55 ml of the spent medium was sent, the pump P-II was stopped. The culturing was continued by performing this operation automatically once every period of time indicated in Table 11. A fresh supply of the culture medium was continuously fed into the culture tank from line A so that the liquid level of the culture tank was constant. On the fourth day after the starting of the culturing and thereafter, the culture fluid containing cells was withdrawn from the culture tank at a rate of 120 ml per day. In the above experiment, $Q/S.\bar{Z}$ was 0.0027 cm/min.; $\theta$ was 30 min., and $\bar{Z}.\theta$ was 2400.

Some of the experimental conditions and the results of the experiment are shown in Table 11.

Table 11

| Culturing time (days) | Time interval for feeding to the centrifugal separator (hours) | Number of feedings of the makeup medium per day | Density of living cells (cells/ml) | Concentration of the antibody in the culture fluid ($\mu$g/ml) |
|---|---|---|---|---|
| 0 | - | 0 | $2.0 \times 10^5$ | 0 |
| 1 | - | 0 | $2.5 \times 10^5$ | 5 |
| 2 | - | 0 | $5.2 \times 10^5$ | 11 |
| 3 | - | 0 | $1.1 \times 10^6$ | 29 |
| 4 | 2.9 | 1.0 | $2.3 \times 10^6$ | 25 |
| 5 | 2.9 | 1.0 | $3.9 \times 10^6$ | 35 |
| 6 | 1.7 | 1.7 | $6.8 \times 10^6$ | 34 |
| 7 | 1.7 | 1.7 | $9.4 \times 10^6$ | 47 |
| 8 | 1.4 | 2.0 | $1.1 \times 10^7$ | 49 |
| 9 | 1.4 | 2.0 | $1.2 \times 10^7$ | 51 |

EXAMPLE 8

(1) Culture apparatus

The same culture apparatus as in Example 1 was used

(2) Culture medium

The same culture medium as in Example 1 was used.
Method and result of culturing
The culture system was sterilized by autoclaving. Then, 1.2 liters of the culture medium sterilized by filtration was fed into the culture tank. Mouse-human hybridoma P3UI cells derived from mouse myeloma P3UI cells were then seeded at a density of 1.1 x $10^5$ cells/ml. These hybridoma cells produce IgG. Oxygen gas containing 5% of carbon dioxide gas was introduced by a blow nozzle B into the culture tank so that the concentration of dissolved oxygen in the culture fluid was maintained at 3 ppm by automatic control. The culture fluid in the culture tank was maintained at 37°C. A marine-type stirring vane was attached to the culture tank, and its stirring speed was 60 rpm.

For 5 days after seeding, batchwise culture was performed. As shown in Table 12, the cell density reached 5.4 x $10^5$/ml on the fifth day after starting the culture. Since this cell density was determined to be the highest density in batchwise culture, and perfusion culture using a centrifugal separator was started. The centrifugal separator charged with the filtration-sterilized culture fluid was driven, and the rotation speed of the centrifugal separator was adjusted so that the centrifuging effect became 80G. The valve X was closed, pump P-II was stopped, and valve Y was opened. In this state, pump P-I was operated to send 200 ml of the culture fluid to the centrifugal separator for 30 minutes at a rate of 6.7 ml/min. The mother liquor separated from the cells in the centrifugal separator was sent to the spent medium vessel AP-3 through line F and stored. In the spent medium vessel AP-3, the cell density was 2.4 x $10^5$ cells/ml. Immediately after the termination of the foregoing operation, valve X was opened, valve Y was closed and pump P-I was stopped. In this state, pump G was driven to send 55 ml of the spent medium to the centrifugal separator at a rate of 11 ml/min. from the vessel AP-3. By this operation, all cells residing in the centrifugal rotor were discharged from the centrifugal separator, and returned to the culture tank AP-1 via the line E. After 55 ml of the spent medium was sent, the pump P-II was stopped. The culturing was continued by performing this operation automatically once every period of time indicated in Table 12. A fresh supply of the culture medium was continuously fed into the culture tank from line A so that the liquid level of the culture tank was constant. On the sixth day after the starting of the culturing and thereafter, the culture fluid containing cells was withdrawn from the culture tank at a rate of 120 ml per day. In the above experiment, $Q/S.\bar{Z}$ was 0.0027 cm/min.; θ was 30 min., and $\bar{Z}.\theta$ was 2400.

Some of the experimental conditions and the results of the experiment are shown in Table 12.

Table 12

| Culturing time (days) | Time interval for feeding to the centrifugal separator (hours) | Number of feedings of the makeup medium per day | Density of living cells (cells/ml) | Concentration of the antibody in the culture fluid (µg/ml) |
|---|---|---|---|---|
| 0 | - | 0 | 1.1 x$10^5$ | 0 |
| 1 | - | 0 | 1.0 x $10^5$ | 2 |
| 2 | - | 0 | 1.9 x $10^5$ | 4 |
| 3 | - | 0 | 3.6 x $10^5$ | 7 |
| 4 | - | 0 | 5.4 x $10^5$ | 10 |
| 5 | 2.9 | 1.0 | 9.8 x $10^5$ | 11 |
| 6 | 2.9 | 1.0 | 1.9 x $10^6$ | 14 |
| 7 | 2.9 | 1.0 | 3.5 x $10^6$ | 16 |
| 8 | 1.7 | 1.7 | 6.4 x $10^6$ | 17 |
| 9 | 1.7 | 1.7 | 9.2 x $10^6$ | 19 |
| 10 | 1.4 | 2.0 | 1.3 x $10^7$ | 20 |
| 11 | 1.4 | 2.0 | 1.2 x $10^7$ | 23 |

EXAMPLE 9

(1) Culture apparatus

The same culture apparatus as in Example 1 was used.

(2) Culture medium

The same culture medium as in Example 1 was used.

Method and result of culturing The culture system was sterilized by autoclaving. Then, 1.2 liters of the culture medium sterilized by filtration was fed into the culture tank. J558L chimera cells which are transformants obtained by introducing human and mouse genes into mouse myeloma J558L as a host were seeded at a density of $1.2 \times 10^5$ cells/ml. These hybridoma cells produce a chimera antibody having a mouse antibody in the V region and a human antibody in a the C region. Oxygen gas containing 5% of carbon dioxide gas was introduced by a blow nozzle B into the culture tank so that the concentration of dissolved oxygen in the culture fluid was maintained at 3 ppm by automatic control. The culture fluid in the culture tank was maintained at 37°C. A marine-type stirring vane was attached to the culture tank, and its stirring speed was 60 rpm to the

For 4 days after seeding, batchwise culture was performed. As shown in Table 13, the cell density reached $6.1 \times 10^5$/ml on the fourth day after starting the reached culture. Since this cell density was determined to be the highest density in batchwise culture, and perfusion culture using a centrifugal separator was started. The centrifugal separator charged with the filtration. The sterilized culture fluid was driven, and the rotation speed of the centrifugal separator was adjusted so that the centrifuging effect became 80G. Then, valve X was closed, pump P-II was stopped, and valve Y was opened. In this state, pump P-I was operated to send 200 ml of the culture fluid to the centrifugal separator for 30 minutes at a rate of 6.7 ml/min. The mother liquor separated from the cells in the centrifugal separator was sent to the spent medium vessel AP-3 through line F and stored. In the spent medium vessel AP-3, the cell density was $2.4 \times 10^5$ cells/ml. Immediately after the termination of the foregoing operation, valve X was opened, valve Y was closed and pump P-I was stopped. In this state, pump G was operated to send 55 ml of the spent medium to the centrifugal separator at a rate of 11 ml/min. from the vessel AP-3. By this operation, all cells residing in the centrifugal rotor were discharged from the centrifugal separator, and returned to the culture tank AP-I via the line E. After 55 ml of the spent medium was sent, the pump P-II was stopped. The culturing was continued by performing this operation automatically once every period of time indicated in Table 13. A fresh supply of the culture medium was continuously fed into the culture tank from line A so that the liquid level of the culture tank was constant. On the fifth day after the starting of the culturing and thereafter, the culture fluid containing cells was withdrawn from the culture tank at a rate of 120 ml per day. In the above experiment, $Q/S.\bar{Z}$ was 0.0027 cm/min.; $\theta$ was 30 min., and $\bar{Z}.\theta$ was 2400.

Some of the experimental conditions and the results of the experiment are shown in Table 13.

Table 13

| Culturing time (days) | Time interval for feeding to the centrifugal separator (hours) | Number of feedings of the makeup medium per day | Density of living cells (cells/ml) | Concentration of the antibody in the culture fluid ($\mu$g/ml) |
|---|---|---|---|---|
| 0 | - | 0 | $1.2 \times 10^5$ | 0 |
| 1 | - | 0 | $1.3 \times 10^5$ | 0.5 |
| 2 | - | 0 | $2.9 \times 10^5$ | 0.9 |
| 3 | - | 0 | $6.1 \times 10^5$ | 2.1 |
| 4 | 2.9 | 1.0 | $1.3 \times 10^6$ | 2.9 |
| 5 | 2.9 | 1.0 | $2.7 \times 10^6$ | 4.6 |
| 6 | 1.9 | 1.5 | $5.5 \times 10^6$ | 5.7 |
| 7 | 1.9 | 1.5 | $9.2 \times 10^6$ | 7.1 |
| 8 | 1.4 | 2.0 | $1.3 \times 10^7$ | 7.3 |
| 9 | 1.4 | 2.0 | $1.4 \times 10^7$ | 9.0 |

**Claims**

1. A method of culturing animal cells, which comprises

(A) subjecting animal cells to suspension culture in a culture tank,

(B) withdrawing a portion of a suspension culture fluid containing the animal cells from the culture tank,

(C) supplying the withdrawn suspension culture fluid to a centrifugal separating device and the centrifugal separating device being operated under the following conditions

$$(1) \qquad \Theta \leq 1.8 \times 10^4,$$

(2)     $\bar{Z} \times \Theta \leq 1.8 \times 10^6$,

(3)     $Q/S.\bar{Z} \leq 5 \times 10^{-3}$,

and

(4)     $5 \leq \bar{Z} \leq 2,000$

wherein

$\Theta$ is the average residence time (seconds) of the animal cells in the centrifugal separating device,

$\bar{Z}$ is a centrifuging effect in the centrifugal separating operation, which is defined by $r\omega^2/g$ in which r is the distance (cm) from the axis of rotation, $\omega$ is the rotating angular speed (radians/sec.), and g is the acceleration of gravity (cm/sec$^2$),

Q is the amount (cm$^3$/s.) of the suspension culture fluid supplied to the centrifugal separating device per unit time, and

S is the sedimentation area (cm$^2$) when the centrifugal force is acting,

(D) withdrawing the separated animal cells from the centrifugal separating device, together with a liquid carrier, by supplying the liquid carrier which

(a) is substantially immiscible with water,

(b) has a higher density than water, and

(c) does not substantially inhibit the growth of the animal cells,

to said centrifugal separating device,

(E) separating animal cells from the liquid carrier, as required, and

(F) recycling at least a portion of the withdrawn animal cells to the culture tank for the step (A).

2.  The method of claim 1 wherein the liquid carrier is supplied together with a culture medium.

3.  The method of claim 1 or 2 wherein the culture medium is a spent medium.

**Patentansprüche**

1.  Verfahren zum Züchten von tierischen Zellen, umfassend:

(A) das Unterwerfen von tierischen Zellen einer Suspensionszüchtung in einem Züchtungstank,

(B) das Entnehmen eines Teils eines Suspensionszüchtungsfluids, welches die tierischen Zellen enthält, aus dem Züchtungstank,

(C) das Zuführen des entnommenen Suspensionszüchtungsfluids zu einer Zentrifugaltrenneinrichtung und Betreiben der Zentrifugaltrenneinrichtung unter den folgenden Bedingungen:

(1)     $\Theta \leq 1,8 \times 10^4$,

(2)     $\bar{Z} \times \Theta \leq 1,8 \times 10^6$,

(3)     $Q/S.\bar{Z} \leq 5 \times 10^{-3}$,

und

$$(4) \qquad 5 \le \bar{Z} \le 2000$$

worin

$\Theta$ die mittlere Verweilzeit (Sekunden) der tierischen Zellen in der Zentrifugaltrenneinrichtung ist,
Z eine Zentrifugierwirkung in dem Zentrifugaltrennvorgang ist, welche definiert ist durch $r\omega^2/g$, worin r der Abstand (cm) von der Rotationsachse ist, $\omega$ die Rotationswinkelgeschwindigkeit (Radiane/s) ist, und g die Schwerkraftbeschleunigung (cm/s$^2$) ist,
Q der Betrag (cm$^3$/s) des Suspensionszüchtungsfluids ist, welcher der Zentrifugaltrenneinrichtung pro Zeiteinheit zugeführt wird, und
S die Sedimentationsfläche (cm$^2$) ist, wenn die Zentrifugalkraft wirkt,

(D) das Entnehmen der abgetrennten tierischen Zellen aus der Zentrifugaltrenneinrichtung zusammen mit einem flüssigen Träger durch Zuführen des flüssigen Trägers, welcher

(a) im wesentlichen unvermischbar mit Wasser ist,

(b) eine höhere Dichte als Wasser hat,

(c) im wesentlichen nicht das Wachstum der tierischen Zellen hemmt bzw. hindert,

zu der Zentrifugaltrenneinrichtung,

(E) das Trennen von tierischen Zellen von dem flüssigen Träger, wenn es erforderlich ist, und

(F) das Rückführen von wenigstens einem Teil der entnommenen tierischen Zellen zu dem Züchtungstank für den Schritt (A).

2.  Verfahren nach Anspruch 1, worin der flüssige Träger zusammen mit einem Züchtungsmedium zugeführt wird.

3.  Verfahren nach Anspruch 1 oder 2, worin das Züchtungsmedium ein verwendetes bzw. verbrauchtes Medium ist.


**Revendications**

1.  Un procédé de culture de cellules animales, qui consiste à

(A) soumettre les cellules animales à une culture en suspension dans une cuve de culture,
(B) soutirer de la cuve de culture une partie d'un liquide de culture en suspension contenant les cellules animales,
(C) introduire dans un dispositif de séparation centrifuge
le liquide de culture en suspension soutiré, le dispositif de séparation centrifuge étant utilisé dans les conditions suivantes :

$$(1) \qquad \theta \le 1{,}8 \times 10^{4},$$

$$(2) \qquad \bar{Z} \times \theta \le 1{,}8 \times 10^{6},$$

$$(3) \qquad Q/S.\bar{Z} \le 5 \times 10^{-3}$$

et

$$(4) \qquad 5 \le \bar{Z} \le 2000$$

où

$\theta$ est le temps de séjour moyen (secondes) des cellules animales dans le dispositif de séparation centrifuge,
Z est un effet de centrifugation dans l'opération de séparation centrifuge, qui est défini par $r\omega^2/g$ où r est la distance (cm) à l'axe de rotation, $\omega$ est la vitesse angulaire de rotation (radians/s) et $g$ est l'accélération de la pesanteur (cm/s$^2$),

Q est la quantité (cm$^3$/s) du liquide de culture en suspension introduit dans le dispositif de séparation centrifuge par unité de temps, et

S est l'aire de sédimentation (cm$^2$) lorsque la force centrifuge est agissante,

(D) retirer du dispositif de séparation centrifuge les cellules animales séparées, en même temps qu'un liquide porteur, en introduisant dans ledit dispositif de séparation centrifuge le liquide porteur qui

(a) est sensiblement non miscible à l'eau,

(b) a une densité supérieure à celle de l'eau, et

(c) n'inhibe sensiblement pas la croissance des cellules animales,

(E) séparer les cellules animales du liquide porteur, comme nécessaire, et

(F) recycler dans la cuve de culture pour l'étape (A) au moins une partie des cellules animales retirées.

2. Le procédé de la revendication 1, dans lequel le liquide porteur est introduit en même temps qu'un milieu de culture.

3. Le procédé de la revendication 1 ou 2, dans lequel le milieu de culture est un milieu usé.

EP 0 480 245 B1

FIG. 1

FIG. 2

FIG. 3

23

# FIG. 4

A

B

# FIG. 5

EP 0 480 245 B1

# FIG. 6

# FIG. 7